# EUROPEAN PATENT APPLICATION

(11) **EP 2 772 270 A1**
(43) Date of publication of application: **03.09.2014**
(21) Application number: 12839933.4
(22) Date of filing: 12.09.2012
(51) Int. Cl.: A61K 47/30, A61K 47/10, A61K 9/107, A61K 9/10

(54) **EMULSIFIED HYDROGEL COMPOSITION AND A PRODUCTION METHOD THEREFOR**

(30) Priority: 11.10.2011 KR 20110103494
(71) Applicant: Genic Co., Ltd., Seocho-gu, Seoul 137-889 (KR)
(72) Inventor: KIM, Sung Jang, Busan 609-401 (KR); PARK, Hyoung-Il, Seoul 134-023 (KR); CHO, Jae Han, Seoul 122-060 (KR); CHOI, Eun Kyoung, Hwaseong-si Gyeonggi-do 445-787 (KR); KWAK, Jae hoon, Daegu 706-050 (KR); LIM, Jae Min, Yongin-si Gyeonggi-do 449-851 (KR); LIM, Hyun Jun, Seoul 139-243 (KR); KIM, Jai Hyun, Seoul 132-806 (KR); KIM, Min Seok, Hwaseong-si Gyeonggi-do 445-747 (KR); YANG, Jin A, Suwon-si Gyeonggi-do 441-850 (KR); YOO, Hyun Oh, Seoul 137-040 (KR); KIM, Jong Chul, Seoul 120-090 (KR)
(74) Representative: Banse & Steglich
(86) International application number: PCT/KR2012/007313
(87) International publication number: WO 2013/055041

(57) **Abstract**

The present invention relates to an emulsified hydrogel composition and to a production method therefor, wherein, by incorporating between 20 and 30 percent by weight of a gelling solution and an emulsion obtained by mixing between 45 and 60 percent by weight of an aqueous component and between 15 and 30 percent by weight of an oil component, it is possible to simultaneously provide the skin with an aqueous fraction and an oil fraction, and it is possible to enhance functionality due to the inclusion of a high content of a dermatologically active component dissolved in the oil component.

## Description

### [Technical Field]

The present invention relates to an emulsified hydrogel composition providing skin lipids and moisture and containing a dermatologically active component dissolved in an oil phase in a high concentration, and a method of manufacturing the same.

### [Background Art]

Skin may be broadly divided into three layers: the epidermis, the dermis, and the subcutis in order from the external layer, and a significant amount of human skin tissue is constantly in direct contact with the external environment in order to protect a human body and has biochemical and physical functions.

Skin keeps sebum and moisture, and a predetermined amount of the oil fraction thereof functions to suppress evaporation of moisture through the skin. A lipid layer on the epidermis of skin functions to prevent water loss from skin, together with the skin lipids. A main component of the lipid layer is ceramide. In Chemical Structural Formula, ceramide has both a hydrophilic group and a lipophilic group and thus functions to prevent moisture contained in skin from evaporating.

As described above, an appropriate amount of the lipids as well as the mositure should be supplied to maintain healthy skin.

Hydrogel mask packs currently on the market are mostly water-soluble and mainly have the function of supplying the mositure, and thus have limitations in use for the age group of people whose skin relatively requires a greater amount of skin lipids replenishment.

### [Disclosure]

### [Technical Problem]

An aspect of the present invention provides a hydrogel composition including an emulsion including an aqueous component and an oil component mixed with each other, and a gelling solution.

Another aspect of the present invention provides a method of manufacturing the hydrogel composition.

### [Technical Solution]

According to an aspect of the present invention, there is provided a hydrogel composition including an emulsion including 45 to 60 wt% of an aqueous component and 15 to 30 wt% of an oil component mixed with each other, and 20 to 30 wt% of a gelling solution.

The aqueous component may include 1 to 20 wt% of a humectant, 78 to 98 wt% of purified water, and 0.1 to 3 wt% of an additive based on a total weight of the aqueous component.

The oil component may include 15 to 25 wt% of an emulsifying agent, 60 to 83 wt% of a skin emollient, and 0.1 to 15 wt% of a skin conditioning agent based on a total weight of the oil component.

The gelling solution may include 0.1 to 20 wt% of a gelling polymer and 80 to 95 wt% of a polyhydric alcohol based on a total weight of the gelling solution.

The humectant may be one or more selected from the group consisting of glycerin, butylene glycol, dipropylene glycol, sodium hyaluronate, betaine, trehalose, propylene glycol, sodium lactate, sorbitol, and 1,2-hexanediol.

The emulsifying agent may be one or more selected from the group consisting of glyceryl stearate, lecithin, sorbitan stearate, sorbitan sesquioleate, sorbitan oleate, sorbitan palmitate, C14-22 alcohol/C12-20 alkyl glucoside, cetearyl alcohol/cetearyl glucoside, polysorbate 60, PEG-100 stearate/glyceryl stearate, PEG-4 oleate, sorbitan olivate, polyglyceryl-3 methylglucose distearate, PEG-8 dimethicone, PEG-10 dimethicone, PEG-9 methyl ether dimethicone, a polyoxyethylene methylpolysiloxane copolymer including PEG-3 dimethicone and PEG-11 methyl ether dimethicone, a poly(oxyethylene-oxypropylene)methylpolysiloxane copolymer including PEG/PPG-20/20 butyl ether dimethicone and PEG/PPG-20/20 dimethicone, an alkyl-chain-silicon-chain branched poly(oxyethylene-oxypropylene)methylpolysiloxane copolymer including cetyl PEG/PPG-10/1-dimethicone and lauryl PEG-9 polydimethylsiloxyethyldimethicone, bis-PEG/PPG-20/5 PEG/PPG-20/5 dimethicone, ABIL® Care XL80 including methoxy PEG/PPG-25/4 dimethicone and caprylic/capric triglyceride, and Lysofix™ including glycerin and glycerin element (soybean) extracts.

The skin emollient may be one or more selected from the group consisting of ester-based oil, hydrocarbon-based oil, wax, natural oil, and silicon oil.

The skin conditioning agent is a component added to the oil component, and one or more selected from the group consisting of retinyl palmitate, tocopherol acetate, ceramide, alpha-bisabolol, coenzyme Q10, and vitamins A, D, and E.

The gelling polymer may be one or more selected from the group consisting of galactomannan, glucomannan, guar gum, locust bean gum, pluronic, agar, algin, carrageenan, xanthan gum, and gellan, and the polyhydric alcohol may be one or more selected from the group consisting of 1,2-ethanediol, 1,2-propanediol, 1,3-propanediol, 3-chloro-1,2-propanediol, 2-chloro-1,3-propanediol, and glycerin.

According to another aspect of the present invention, there is provided a method of manufacturing a hydrogel composition, which includes heating 45 to 60 wt% of an aqueous component and 15 to 30 wt% of an oil component to a temperature of 70 to 80°C, mixing the heated oil component and aqueous component at a temperature of 70 to 80°C to manufacture an emulsion, and adding 20 to 30 wt% of a gelling solution to the emulsion to perform mixing, and then performing stirring at a temperature of 70 to 80°C for 20 to 40 minutes.

### [Advantageous Effects]

A hydrogel composition of the present invention provides both lipids and moisture to skin to reduce transepidermal water loss.

Further, the hydrogel composition of the present invention contains a dermatologically active component dissolved in an oil component in a high concentration to further improve functionality.

### [Best Mode]

The present invention relates to an emulsion hydrogel composition providing both an aqueous fraction and an oil fraction to skin to reduce transepidermal water loss, and a method of manufacturing the same.

Hereinafter, the present invention will be described in detail.

The hydrogel composition includes an emulsion including an aqueous component and an oil component mixed with each other, and a gelling solution, and other additives may be further added according to the use.

The aqueous component includes purified water, a humectant, and an additive, which are required to supply the aqueous fraction and form a hydrogel. The content of the aqueous component is 45 to 60 wt% and preferably 50 to 58 wt% based on the total weight of the hydrogel composition.

When the content of the aqueous component is less than 45 wt%, viscosity of the gel is increased when the hydrogel is manufactured, and thus sufficient fluidity cannot be ensured during a manufacturing process, and when the content is greater than 60 wt%, there is no difficulty in manufacturing of the hydrogel, but the emulsion is not formed when the aqueous component is mixed with the oil component.

Purified water may be used in a content of 78 to 98 wt% based on the total weight of the aqueous component.

The humectant supplies moisture to skin, and specific examples may include one or two or more selected from the group consisting of glycerin, butylene glycol, dipropylene glycol, sodium hyaluronate, betaine, trehalose, propylene glycol, sodium lactate, sorbitol, and 1,2-hexanediol.

The content of the humectant is 1 to 20 wt% and preferably 2 to 18 wt% based on the total weight of the aqueous component. When the content of the humectant is less than 1 wt%, a humectation function may be reduced, and when the content is greater than 20 wt%, stickiness may occur to affect viscosity and properties of the hydrogel composition.

Examples of the additive may include one or two or more selected from the group consisting of a skin conditioning agent improving dried or damaged skin and providing flexibility of skin, a sequestering agent removing iron and copper ions affecting acidification of the hydrogel composition, and a preservative for storing the hydrogel composition over an extended period of time.

The content of the additive is 0.1 to 3 wt% and preferably 0.5 to 2 wt% based on the total weight of the aqueous component.

Examples of the skin conditioning agent added to the aqueous component may include one or two or more selected from the group consisting of arbutin, niacinamide, adenosine, ascorbyl glucoside, and natural extracts. The natural extracts may be selected from the group consisting of extracts of aloe, green tea, ginseng, red ginseng, pyroligneous liquor, pine needles, ginkgo leaves, propolis, mulberry leaves, silkworms, snail slime, Kakadu plum, Camu camu, Assai palm, squalane, caviar, broccoli, blueberries, Witch-hazel, acerola, chlorella, mangosteen, guava, spirulina, salmon roe, Ecklonia cava, giant kelp, kelp, Portulaca oleracea, green laver, agar, roots of mulberry trees, raspberries, wild berries, Hijikia fusiforme, sargassum, edelweiss, chamomile, lavender, peppermint, eucalyptus, lemon balm, oregano, tea tree, skullcap, Houttuynia cordata, sea buckthorn, citron, albumin, egg yolk, and milk proteins.

Examples of the sequestering agent may include one or two or more selected from the group consisting of a disodium EDTA (disodium ethylenediamine tetraacetic acid), a trisodium EDTA (trisodium ethylenediamine tetraacetic acid), and a nitrilotriacetic acid.

Examples of the preservative may include one or two or more selected from the group consisting of methylparaben, propylparaben, phenoxyethanol, and ethylparaben.

Since the oil component includes the emulsifying agent, the skin emollient, and the skin conditioning agent to contain the oil fraction in a higher content as compared to a water-soluble hydrogel, transepidermal water loss may be reduced. The content of the oil component is 15 to 30 wt% and preferably 20 to 30 wt% based on the total weight of the hydrogel composition.

When the content of the oil component is less than 15 wt%, the emulsion is not formed when the oil component is mixed with the aqueous component, and when the content is greater than 30 wt%, viscosity is increased during gelling, and thus drying is performed during manufacturing, and stability of the gel is reduced.

The emulsifying agent is used to easily mix the aqueous component and the oil component, and specific examples may include one or two or more selected from the group consisting of glyceryl stearate, lecithin, sorbitan stearate, sorbitan sesquioleate, sorbitan oleate, sorbitan palmitate, C14-22 alcohol/C12-20 alkyl glucoside, cetearyl alcohol/cetearyl glucoside, polysorbate 60, PEG-100 stearate/glyceryl stearate, PEG-4 oleate, sorbitan olivate, polyglyceryl-3 methylglucose distearate, PEG-8 dimethicone, PEG-10 dimethicone, PEG-9 methyl ether dimethicone, a polyoxyethylene methylpolysiloxane copolymer including PEG-3 dimethicone and PEG-11 methyl ether dimethicone, a poly(oxyethylene-oxypropylene)methylpolysiloxane copolymer including PEG/PPG-20/20 butyl ether dimethicone and PEG/PPG-20/20 dimethicone, an alkyl-chain-silicon-chain branched poly(oxyethylene-oxypropylene)methylpolysiloxane copolymer including cetyl PEG/PPG-10/1-dimethicone and lauryl PEG-9 polydimethylsiloxyethyldimethicone, bis-PEG/PPG-20/5 PEG/PPG-20/5 dimethicone, ABIL® Care XL80 including methoxy PEG/PPG-25/4 dimethicone and caprylic/capric triglyceride, and Lysofix™ including glycerin and glycerin element (soybean) extracts.

The content of the emulsifying agent is 15 to 25 wt% and preferably 18 to 23 wt%, based on the total weight of the oil component. When the content of the emulsifying agent is less than 15 wt%, an aqueous phase and an oil phase are not mixed but separated, and thus the emulsion is not formed, and when the content is greater than 25 wt%, a gelling rate is increased during mass production, and thus stability of the gel is reduced.

The skin emollient softens skin, and specific examples may include one or two or more selected from the group consisting of an ester-based oil such as caprylic/caprictri glyceride, butyleneglycol dicaprylate/dicaprate, triethylhexanoin, cetylethyl hexanoate, isopropyl myristate, and octyldodecanol; a hydrocarbon-based oil such as liquid isoparaffin, vaseline, paraffin, and hydrogenated polydecene; a wax such as microcrystalline wax, beeswax, and lanolin wax; a natural oil such as squalene, mineral oil, Jojoba oil, avocado oil, almond oil, olive oil, cacao butter, sesame oil, wheat germ oil, safflower oil, sheabutter, camellia oil, castor oil, grape seed oil, green tea seed oil, macadamia nut oil, coconut oil, rose hip oil, hardened oil, argan oil, sea buckthorn oil, argania spinosa kernel oil, and lavender oil; and a silicon oil such as cyclomethicone, phenyltrimethicone, cyclopentasiloxane, and dimethicone.

The content of the skin emollient is 60 to 83 wt% and preferably 65 to 80 wt%. When the content of the skin emollient is less than 60 wt%, it is difficult to form the emulsion and an oil fraction content is small after the hydrogel is manufactured, and thus a target reduction in transepidermal water loss cannot be expected, and when the content is greater than 83 wt%, the emulsion is not formed and the amount of aqueous component is relatively reduced, and thus it is difficult to manufacture the hydrogel.

Examples of the skin conditioning agent added to the oil component include one or more selected from the group consisting of retinyl palmitate, tocopherol acetate, ceramide, alpha-bisabolol, coenzyme Q10, and vitamins A, D, and E.

The content of the skin conditioning agent is 0.1 to 15 wt% and preferably 1 to 12 wt%. When the content of the skin conditioning agent is less than 0.1 wt%, target functionality is not ensured, and when the content is greater than 15 wt%, side effects such as skin stimulation occur.

The gelling solution is added to the manufactured emulsion in a content of 20 to 30 wt% and preferably 25 to 28 wt% based on the total weight of the hydrogel composition to manufacture the hydrogel composition.

When the content of the gelling solution is less than 20 wt%, it is difficult to perform gelling with the emulsion, and when the content is greater than 30 wt%, the gel is hardened, and thus it is difficult to release an effective component of the emulsion to skin.

The gelling solution includes a gelling polymer and a polyhydric alcohol.

Specific examples of the gelling polymer may include one or two or more selected from the group consisting of galactomannan, glucomannan, guar gum, locust bean gum, pluronic, agar, algin, carrageenan, xanthan gum, and gellan.

The content of the gelling polymer is 0.1 to 20 wt% and preferably 0.5 to 15 wt%. When the content of the gelling polymer is less than 0.1 wt%, the form of the gel can be maintained but the strength of the gel is high and flexibility is reduced, and thus close contact properties with skin are reduced, and when the content is greater than 20 wt%, it is difficult to maintain the form because the gel droops, it is difficult to manufacture the hydrogel due to reduced elasticity, and adhesion to skin is reduced.

Further, the polyhydric alcohol dissolves the gelling polymer and provides flowability to the hydrogel to allow the hydrogel to come into close contact with skin and allow the emulsion to permeate skin.

Specific examples of the polyhydric alcohol may include one or two or more selected from the group consisting of 1,2-ethanediol, 1,2-propanediol, 1,3-propanediol, 3-chloro-1,2-propanediol, 2-chloro-1,3-propanediol, and glycerin, but preferably, glycerin is used.

The content of the polyhydric alcohol is 80 to 95 wt% and preferably 85 to 95 wt% based on the total weight of the gelling solution. When the content of the polyhydric alcohol is less than 80 wt%, flowability cannot be provided to the hydrogel, and when the content is greater than 95 wt%, the stickiness is increased.

Further, one or two or more additives selected from the group consisting of a pH adjuster, an antioxidant, a whitening agent, a wrinkle functional agent, and an antimicrobial may be added to the hydrogel composition in a content of 0.01 to 5 wt% based on the total weight of the hydrogel composition according to the use. When the content of the additive is less than 0.01 wt%, the function of the additive cannot be ensured, and when the content is greater than 5 wt%, the additive may affect properties of the hydrogel composition.

The additive may be added to the emulsion before added to the gelling solution, to the gelling solution before mixed with the emulsion, or to a solution including the emulsion and the gelling solution mixed with each other. For example, the pH adjuster is added to the gelling solution before mixed with the emulsion, and the antioxidant, the whitening agent, the wrinkle functional agent, and the antimicrobial are added to the emulsion before added to the gelling solution or the solution including the emulsion and the gelling solution mixed with each other.

Specific examples of the pH adjuster may include one or two or more selected from the group consisting of a citric acid, potassium hydroxide, and arginine.

Further, the present invention provides a method of manufacturing the hydrogel composition by using the hydrogel composition.

The hydrogel composition is manufactured by the method including heating the aqueous component and the oil component in operation S110, mixing the heated oil component and aqueous component to manufacture an emulsion solution in operation S120, and mixing the emulsion solution and the gelling solution in operation S130.

During operation S110, the humectant, purified water, and the additive are added to a vessel, and then heated to a temperature of 70 to 80°C while stirred (aqueous component), and the emulsifying agent, the skin emollient, and the skin conditioning agent are added to another vessel, and then heated to a temperature of 70 to 80°C while stirred (oil component). When the temperature of each of the aqueous component and the oil component is less than 70°C, the aqueous component and the oil component are not easily mixed during operation S120, and when the temperature is greater than 80°C, evaporation may easily occur due with oils having different boiling points, and stability of the emulsifying agent or the natural component is reduced.

During operation S120, the aqueous component and the oil component prepared during operation S110 are mixed at a temperature of 70 to 80°C and then emulsified by an emulsifying machine to manufacture the emulsion. When a mixing temperature of the aqueous component and the oil component is less than 70°C, the aqueous component and the oil component are not easily mixed, and thus the emulsion cannot be manufactured, and when the temperature is greater than 80°C, stability of a material added to the aqueous component and the oil component is reduced.

During operation S130, the gelling solution is added to the emulsion manufactured during operation S120, mixed, and stirred for 20 to 40 minutes while maintained at a temperature of 70 to 80°C. When a stirring time is less than 20 minutes, the hydrogel composition is not sufficiently gelled, and when the stirring time is greater than 40 minutes, the gel may be hardened and densified, and thus it may be difficult to provide the effective component of the emulsion to skin.

After operation S130, the additive such as the pH adjuster, the antioxidant, the whitening agent, the wrinkle functional agent, and the antimicrobial may be added according to the use.

A hydrogel may be manufactured using the manufactured hydrogel composition according to a method of manufacturing the hydrogel used in the art. For example, the manufactured hydrogel composition may be applied on a sheet, cooled, and molded to provide a target form, thus manufacturing the hydrogel. Alternatively, the hydrogel composition may be poured on a mold molded to have a target form to manufacture the hydrogel without the sheet.

### [Mode for Invention]

Examples will be given hereinafter to help understanding of the present invention, but the following Examples have been described in an illustrative manner, and it is to be understood that various modifications and amendments will be apparent to those skilled in the art without departing from the spirit of the invention, and the modifications and the amendments naturally fall into the scope of the appended claims.

### Preparation Example 1. Preparation of the aqueous component

89 . 78 wt% of purified water, 0.03 wt% of the disodium EDTA, 0.32 wt% of methylparaben, 1.87 wt% of betaine, and 8 wt% of butylene glycol were added to the vessel, and then stirred to prepare the aqueous component.

### Preparation Example 2. Preparation of the oil component

7.4 wt% of glyceryl stearate, 60.9 wt% of polysorbate, 6.4 wt% of sorbitan sesquioleate, 33.16 wt% of caprylic/caprictriglyceride, 15 wt% of dimethicone, 15 wt% of cyclopentasiloxane, 7.02 wt% of tocopheryl acetate, and 7.02 wt% of retinyl palmitate were added to the vessel, and then stirred to prepare the oil component.

### Preparation Example 3. Gelling solution

87.8 wt% of glycerin, 3.51 wt% of carrageenan, and 8.69 wt% of locust bean gum were added, and then stirred to prepare the gelling solution.

### Example 1

The aqueous component prepared in Preparation Example 1 was heated to 75°C, and the oil component prepared in Preparation Example 2 was heated to a temperature of 75°C. 55 wt% of the heated aqueous component and 20 wt% of the heated oil component were mixed, and then emulsified by the homomixer at a temperature of 75°C for 5 minutes to manufacture the emulsion. 25 wt% of the gelling solution was added to the manufactured emulsion, and then stirred for 30 minutes while maintained at a temperature of 75°C to manufacture the hydrogel composition.

In this case, a weight ratio of the aqueous component and the oil component was 2.75 : 1, and a weight ratio of the emulsion and the gelling solution was 3 : 1.

### Example 2

The same procedure as Example 1 was performed to manufacture the hydrogel composition, except that the content of the aqueous component was 45 wt% and the content of the oil component was 30 wt%.

In this case, a weight ratio of the aqueous component and the oil component was 1.5 : 1, and a weight ratio of the emulsion and the gelling solution was 3 : 1.

### Example 3

The same procedure as Example 1 was performed to manufacture the hydrogel composition, except that the content of the aqueous component was 55 wt%, the content of the oil component was 20 wt%, and the content of the gelling solution was 20 wt%.

In this case, a weight ratio of the aqueous component and the oil component was 2.75 : 1, and a weight ratio of the emulsion and the gelling solution was 3.75 : 1.

### Example 4

The same procedure as Example 1 was performed to manufacture the hydrogel composition, except that the content of the aqueous component was 45 wt%, the content of the oil component was 30 wt%, and the content of the gelling solution was 20 wt%.

In this case, a weight ratio of the aqueous component and the oil component was 1.5 : 1, and a weight ratio of the emulsion and the gelling solution was 3.75 : 1.

### Comparative Example 1

The same procedure as Example 1 was performed to manufacture the hydrogel composition, except that the oil component was not added and the content of the aqueous component was 75 wt%.

### Comparative Example 2

The same procedure as Example 1 was performed to manufacture the hydrogel composition, except that the gelling solution was first added to the aqueous component and the oil component was then added.

### Comparative Example 3

The same procedure as Example 1 was performed to manufacture the hydrogel composition, except that the content of the aqueous component was 40 wt%, the content of the oil component was 45 wt%, and the content of the gelling solution was 15 wt%.

### Comparative Example 4

The same procedure as Example 1 was performed to manufacture the hydrogel composition, except that the content of the aqueous component was 30 wt%, the content of the oil component was 30 wt%, and the content of the gelling solution was 40 wt%.

### Test Example

Physical properties of the hydrogel manufactured using the hydrogel composition manufactured in the Examples and the Comparative Examples were measured, and the results are described in the following Table 1.
1. Degree of humectation: The manufactured hydrogel was cut into a circular form having the diameter of 10 mm, the initial weight was measured, the circular hydrogel adhered to the internal sides of the upper arms of twenty healthy women for 30 minutes and was then detached, the weight was measured, and a difference in weight was obtained. The plastic having the same size as the hydrogel was covered and fixed in order to prevent the aqueous fraction from evaporating.
Further, in order to measure the humectation ability of the portion to which the hydrogel adhered, the water retention amount (AU) was non-invasively measured by using Corneometer CM 825 (Courage and Khazaka, Germany) as equipment measuring capacitance in an in vivo state, and the water loss (g/m²h) was obtained as the average value of twenty women by using a Tewarmeter TM210 (Courage and Khazaka, Germany) as a measuring device to measure transepidermal water loss (hereinafter, referred to as 'TEWL').
2. Degree of stimulation to skin: The hydrogel was cut into a circular form having the diameter of 10 mm, adhered to the internal sides of the upper arms of twenty healthy women, and was detached after 24 hours, and the state of skin was observed by the naked eye and evaluated according to the following evaluation standard.
⊚: No stimulation
○: Weak stimulation
□: Erythema
Δ: Erythema and edema
X: Erythema, edema, and blistering

3. Stability (discoloration, gel fluidization, drying): While the hydrogel was left in the air at a temperature of 25°C, the physical change of the gel was measured by the naked eye after 60 minutes, 5 hours, 10 hours, and 24 hours, and evaluated according to the following evaluation standard.
Ⓞ: Maintenance of properties
○: Discoloration, gel fluidization, and drying in an area of less than 10% of the total area
□: Discoloration, gel fluidization, and drying in an area of less than 30% of the total area
Δ: Discoloration, gel fluidization, and drying in an area of less than 50% of the total area
X: Discoloration, gel fluidization, and drying in an area of less than 80% of the total area

**[Table 1]**

| Classification | | Example 1 | Example 2 | Example 3 | Example 4 | Comparative Example 1 | Comparative Example 2 | Comparative Example 3 | Comparative Example 4 |
|---|---|---|---|---|---|---|---|---|---|
| degree of humectation | Weight (g) difference | 0.3 | 0.35 | 0.2 | 0.21 | 0.2 | 0.21 | 0.28 | 0.27 |
| | Initial corneo (AU) | 32.0 | 29.0 | 28.7 | 30.3 | 28.2 | 27.3 | 27.2 | 24.0 |
| | Corneo after 30 minutes (AU) | 35.5 | 34.1 | 33.4 | 34.1 | 33.2 | 28.3 | 29.1 | 27.4 |
| | Initial TEWL (g/m²h) | 7.0 | 6.5 | 6.5 | 6.5 | 6.0 | 6.3 | 6.2 | 6.3 |
| | TEWL after 30 minutes (g/m²h) | 6.7 | 6.1 | 6.3 | 6.2 | 4.3 | 4.5 | 4.5 | 4.1 |
| Degree of stimulation to skin | | ⊚ | ⊚ | ⊚ | ⊚ | ⊚ | No formulation | ○ | ○ |
| Stability | 60 minutes | ⊚ | ⊚ | ⊚ | ⊚ | ⊚ | No formulation | ○ | ○ |
| | 5 hours | ⊚ | ⊚ | ⊚ | ⊚ | ○ | No formulation | Δ | Δ |
| | 10 hours | ⊚ | ⊚ | ⊚ | ⊚ | Δ | No formulation | X | X |
| | 24 hours | ⊚ | ⊚ | ⊚ | ⊚ | X | No formulation | X | X |

As shown in Table 1, it was confirmed that Examples 1 to 4 of the present invention had the excellent degree of humectation, degree of stimulation to skin, and stability.

On the other hand, the degree of humectation, the degree of stimulation to skin, and stability of Comparative Examples 1 to 4 were not excellent.

4. Percutaneous absorption evaluation: Percutaneous absorption to oil-soluble tocopheryl acetate was evaluated. That is, the in vitro skin permeation test was performed by using skin of a hairless mouse. The samples of Examples 1, 2, and 3 and Comparative Example 1 were applied in the amount of 48 micro-liters on the surface of skin having the diameter of 15.0 mm, the amount of tocopheryl acetate permeating by using percutaneous absorption equipment (Hanson Microette) was weighed after 24 hours by liquid chromatography, and the results are described in Table 2. As described above, it can be seen that percutaneous absorption efficiency of the Examples is better than that of the Comparative Examples.

**[Table 2]**

| Sample | Example 1 | Example 2 | Example 3 | Comparative Example 3 |
|---|---|---|---|---|
| Transmittance | 15.3% | 16.1% | 15.5% | 7.2% |

## Claims

1. An emulsified hydrogel composition comprising:
an emulsion comprising 45 to 60 wt% of an aqueous component and 15 to 30 wt% of an oil component mixed with each other; and
20 to 30 wt% of a gelling solution.

2. The emulsified hydrogel composition of claim 1, wherein the aqueous component comprises 1 to 20 wt% of a humectant, 78 to 98 wt% of purified water, and 0.1 to 3 wt% of an additive based on a total weight of the aqueous component.

3. The emulsified hydrogel composition of claim 1, wherein the oil component comprises 15 to 25 wt% of an emulsifying agent, 60 to 83 wt% of a skin emollient, and 0.1 to 15 wt% of a skin conditioning agent based on a total weight of the oil component.

4. The emulsified hydrogel composition of claim 1, wherein the gelling solution comprises 0.1 to 20 wt% of a gelling polymer and 80 to 95 wt% of a polyhydric alcohol based on a total weight of the gelling solution.

5. The emulsified hydrogel composition of claim 2, wherein the humectant is one or more selected from the group consisting of glycerin, butylene glycol, dipropylene glycol, sodium hyaluronate, betaine, trehalose, propylene glycol, sodium lactate, sorbitol, and 1,2-hexanediol.

6. The emulsified hydrogel composition of claim 3, wherein the emulsifying agent is one or more selected from the group consisting of glyceryl stearate, lecithin, sorbitan stearate, sorbitan sesquioleate, sorbitan oleate, sorbitan palmitate, C14-22 alcohol/C12-20 alkyl glucoside, cetearyl alcohol/cetearyl glucoside, polysorbate 60, PEG-100 stearate/glyceryl stearate, PEG-4 oleate, sorbitan olivate, polyglyceryl-3 methylglucose distearate, PEG-8 dimethicone, PEG-10 dimethicone, PEG-9 methyl ether dimethicone, a polyoxyethylene methylpolysiloxane copolymer comprising PEG-3 dimethicone and PEG-11 methyl ether dimethicone, a poly(oxyethylene-oxypropylene)methylpolysiloxane copolymer comprising PEG/PPG-20/20 butyl ether dimethicone and PEG/PPG-20/20 dimethicone, an alkyl-chain-silicon-chain branched poly(oxyethylene-oxypropylene)methylpolysiloxane copolymer comprising cetyl PEG/PPG-10/1-dimethicone and lauryl PEG-9 polydimethylsiloxyethyldimethicone, bis-PEG/PPG-20/5 PEG/PPG-20/5 dimethicone, ABIL® Care XL80 comprising methoxy PEG/PPG-25/4 dimethicone and caprylic/capric triglyceride, and Lysofix^{™} comprising glycerin and glycerin element (soybean) extracts.

7. The emulsified hydrogel composition of claim 3, wherein the skin emollient is one or more selected from the group consisting of ester-based oil, hydrocarbon-based oil, wax, natural oil, and silicon oil.

8. The emulsified hydrogel composition of claim 3, wherein the skin conditioning agent added to the oil component is one or more selected from the group consisting of retinyl palmitate, tocopherol acetate, ceramide, alpha-bisabolol, coenzyme Q10, and vitamins A, D, and E.

9. The emulsified hydrogel composition of claim 4, wherein the gelling polymer is one or more polysaccharide polymers selected from the group consisting of galactomannan, glucomannan, guar gum, locust bean gum, pluronic, agar, algin, carrageenan, xanthan gum, and gellan.

10. The emulsified hydrogel composition of claim 4, wherein the polyhydric alcohol is one or more selected from the group consisting of 1,2-ethanediol, 1,2-propanediol, 1,3-propanediol, 3-chloro-1,2-propanediol, 2-chloro-1,3-propanediol, and glycerin.

11. A method of manufacturing an emulsified hydrogel composition, the method comprising:
heating 45 to 60 wt% of an aqueous component and 15 to 30 wt% of an oil component to a temperature of 70 to 80°C;
mixing the heated oil component and aqueous component at a temperature of 70 to 80°C to manufacture an emulsion; and
adding 20 to 30 wt% of a gelling solution to the emulsion to perform mixing, and then performing stirring at a temperature of 70 to 80°C for 20 to 40 minutes.
